# EUROPÄISCHE PATENTSCHRIFT

(11) **EP 0 498 899 B1**
(45) Veröffentlichungstag und Bekanntmachung des Hinweises auf die Patenterteilung: **07.10.1998**
(21) Anmeldenummer: 90124375.8
(22) Anmeldetag: 17.12.1990
(51) Int. Cl.: A61K 31/40, A61K 38/55

(54) **Verwendung von N-(Mercaptoacyl)aminosäuren als dopaminergen und gonadotropen Arzneimitteln**
Use of N-(mercaptoacyl)-amino acids as dopaminergic und gonadoptropic medicaments
Utilisation de N-(mercaptoacyl)-acides aminés comme médicaments dopaminergiques et gonadotropiques

(43) Veröffentlichungstag der Anmeldung: 19.08.1992
(73) Patentinhaber: Weth, Gosbert Dr. med., Dr. rer. nat., 95346 Stadtsteinach (DE)
(72) Erfinder: Weth, Gosbert Dr. med., Dr. rer. nat., 95346 Stadtsteinach (DE)

(56) Entgegenhaltungen:
- DE-A- 3 932 749
- Lombardi, C. et al.: JOURNAL OF BIOLOGICAL REGULATIONS AND HOMEOSTATICS AGENTS, Band. 3, Nr. 3, 1989, Seiten 128-130
- ANTGIOLOGY, Band 41, Nr. 5, Mai 1990, Seiten 377-381, New York, US; I. SAITO et al.: "Effect of captopril on plasma prolactin in patients with essential hypertension"
- PROLACTIN, BASIC AND CLINICAL CORRELATES, Band 1, 1985, Seite 53-57, Liviana Press, Padova, IT; C. DENEF: "Paracrine interaction in anterior pituitary"
- J.E.F. REYNOLDS et al, (HrsG): "Martindale, the extra pharmacopoeia", Ed. 29, 1989, Seiten 468-472, London, GB
- THE JOURNAL OF CLINICAL ENDOCRINOLOGY & METABOLISM, Band 64, Nr. 4, April 1987, Seiten 713-717 The Endocrine Society, US; W.B. MALARKEY et al.: "Angiotensin II promotes prolactin release from normal human anterior pituitary dependent manner"

## Beschreibung

Die Erfindung bezieht sich auf die Verwendung von N-(Mercaptoacyl)-aminosäuren und deren Salze als Arzneimittel.

Erhöhtes Adrenalin ist mitverantwortlich für den Bluthochdruck. N-(Mercaptoacyl)-aminosäuren (ACE-Hemmer) senken bekanntlich den Bluthochdruck durch die Hemmung der Bildung von Angiotensin II. Sie werden deshalb als blutdrucksenkendes Mittel eingesetzt. Dabei wird eine leichte Reduktion des Noradrenalins diskutiert. Noradrenalin wird jedoch aus Dopamin gebildet und somit wurde bisher angenommen, daß es neben der Bildung von Noradrenalin auch zu einer Senkung des Dopamin kommt.

Ferner ist bekannt, daß die Prolaktinfreisetzung gehemmt wird durch den Stoff PIF (Prolaktin-Inhibitor-Faktor), der als Dopamin identifiziert wurde (FORTH, HENSCHLER, "Allgemeine und spezielle Pharmakologie und Toxikologie, Bibliograph. wissenschaftl. Institut, 1987, S. 124-139, S. 160 u. S. 140). Bisher wurden dopaminerge Substanzen, z.B. Dopa bei Parkinsonpatienten eingesetzt.

Es ist bekannt, daß gonadotrope Hormone nur im Gehirn gebildet werden. Die gonadotrope Wirkung kann durch die bekannten gonadotropen Hormone FSH (Follikelstimulierendes Hormon) und LH (Luteotropes oder auch Luteinisierungs-Hormon genannt) festgestellt werden.

Diese Hormone stimulieren die Gonaden, steigern den Testosteronspiegel bei Männern und die Gestagene und Oestrogene bei Frauen.

In der Hypophyse befinden sich die FSH und LH produzierenden gonadotropen Zellen (Funktion und Mechanismus in "Butt, W.R.: The chemistry of gonadotropins. Thomas, Springfield/III.1967).

Es wurde gefunden, daß N-(Mercaptoacyl)-aminosäuren durch Freisetzung von Dopamin zu einer Durchblutungssteigerung, sowohl peripher als auch zentral, führen. Sie zeigen aufgrund ihrer dopaminergen und gonadotropen Wirkung einen durchblutungsfördernden Effekt.

Mit der erfindungsgemäßen Lösung kommt es zu einem hochsignifikaneten Anstieg von Dopamin, was bei dem bekannten Hintergrund überraschend ist. Dopamin kann bei Durchblutungsstörungen bei Patienten mit niedrigem Blutdruck zur Kreislaufstabilisierung eingesetzt werden. In Untersuchungen konnte gezeigt werden, daß bei Patienten mit einem niedrigen Blutdruck es nicht zu einer weiteren Senkung des Blutdrucks kam, aber zu einer vermehrten peripheren und zentralen Durchblutung.

Desweiteren wurde in tierexperimentellen Untersuchungen gefunden, daß die durch Noradrenalin bedingte Minderung der Durchblutung durch gleichzeitige Infusion mit N-(Mercaptoacyl)-aminosäuren deutlich gesteigert wird und die α-spastischen Effekte des Noradrenalins durch N-(Mercaptoacyl)-aminosäuren aufgehoben werden. Dies beweist eindeutig die Duchblutungssteigerung dieser Verbindungen.

Da die mit der Erfindung erreichte dopaminerge Wirkung auch cerebral nachweisbar ist, ist die mit der Erfindung erreichte Wirkung auch im Gehirn durch Neurotransmitterveränderungen feststellbar. Die Neurotransmitterveränderungen wurden mit der Methode von Dr. Dr. G. Weth, Dissertation, Würzburg, 1987, S. 123-138, bestimmt.

In Untersuchungen konnte die gonadotrope Wirkung nachgewiesen werden, die ein weiterer Beweis für den cerebral durchblutungssteigernden Effekt ist.

Gemäß der Erfindung geeignete dopaminerge und gonadotrope N-(Mercaptoacyl)-aminosäuren entsprechen der allgemeinen Formel 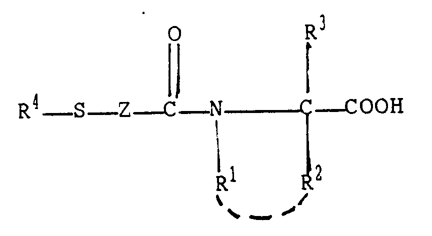 worin
- R¹: Wasserstoff, niedriges Alkyl oder Carboxyl,
- R²: Wasserstoff, niedriges Alkyl, Phenyl, Aralkyl, Imidazolylalkyl oder Indolylalkyl, die substituiert sein können durch niedriges Alkyl, Hydroxy, Alkylhydroxy, Methylendioxy, Mercapto, Amido, Guanidino oder Carboxy,
- R¹ und R²: gemeinsam einen Pyrrolidin-, Piperidin- oder Thiazolidinring vervollständigen können und jeder Ring substituiert sein kann durch niedriges Alkyl, Aralkyl, Phenyl, Furyl, Thienyl, Pyridyl oder Naphthyl, die ihrerseits substituiert sein können durch ein niedriges Alkyl, Hydroxyniedrigalkyl, Mercaptoniedrigalkyl, Hydroxy, Niedrigalkoxy, Alkylendioxy, Halogen, Nitro, Amino, Niedrigalkylamino oder Acylamino,
- R³: Wasserstoff oder Methyl,
- R⁴: Wasserstoff oder Alkyl,
- Z: geradkettiges oder verzweigtes Alkylen mit 1 bis 3 Kohlenstoffatomen bedeuten
und deren Salze.

Besonders geeignete N-(Mercaptoacyl)-aminosäuren sind (2R,4R)-2-(2-Hydroxyphenyl)-3-(3-mercaptopropionyl)-4-thiazolidincarbonsäure und 1-[(2S)-3-Mercapto-2-methyl-propionyl]-2-prolin. Mit besonderem Vorteil werden im Handel erhältliche Verbindungen, wie z.B. Captopril, verwendet.

Die erfindungsgemäß verwendeten dopaminergen und durhcblutungsfördernden Verbindungen können als Wirkstoff in Tabletten, als Granulat oder als Injektionspräparat verabreicht werden. Besondere Vorteile der erfindungsgemäß verwendeten Verbindungen liegen in der kostengünstigen Herstellung und der guten Verträglichkeit beim Patienten.

Es wurde gefunden, daß die erfindungsgemäß verwendeten N-(Mercaptoacyl)-aminosäuren zu einer erhöhten Freisetzung von Dopamin führen. Durch die gonadotrope Wirkung wird ein stark durchblutungsfördernder Effekt erreicht. All dies konnte durch pharmakologische Tests bewiesen werden.

Die Dopaminbestimmung wurde nach G. Weth, "Das Verhalten der Neurotransmitter....", Dissertation, Würzburg, 1987, S. 123-138, durchgeführt.

Für die Untersuchungen wurden 2x12 Patienten (Alter 75±10 Jahre), die unter Durchblutungsstörungen, sowohl peripheren als auch zentralen, litten, herangezogen. Bei diesen Patienten wurden sowohl vor der Untersuchung, als auch 15, 30, 60, und 120 Minuten nach der Untersuchung Dopamin bestimmt. Dabei zeigte sich, daß es bei diesen Patienten bei Gabe von entweder 12,5 oder 25 mg N-(Mercaptoacyl)-aminosäure sublingual es innerhalb von 15 min zu einem hochsignifikanten Anstieg des Dopamins kam (RR lag zwischen 100/70 und 100/110; MW=130/90).

Die Bestimmung der Dopaminwerte wurde wie folgt vorgenommen. In angegebenen Zeitabschnitten von 15, 30, 60 und 120 Minuten wurden dem Patienten je 5 ml Blut entnommen und in speziell präparierte Vakuum-Container, die Glutathion und EDTA enthielten, gegeben. Das Blut wurde sofort auf 2 °C abgekühlt und bei 2 °C zentrifugiert. Das überstehende Blutplasma wurde bis zur Dopaminbestimmung bei minus 80 °C tiefgefroren.

In den Tabellen sind die Ergebnisse der vorstehend angeführten pharmakologischen Untersuchungen zusammengestellt. Die Dimensionsangaben betragen für Dopamin pg/ml bzw. ng/l.

Tabelle 1 zeigt die statistische Auswertung der Dopaminwerte nach Gabe von 25 mg Captopril.

**Tabelle 1**

| Dopamin | Zeit | MW (pg/ml) | Str. | N | P (T) |
|---|---|---|---|---|---|
| Dop. | 0 | 110 | 52 | 12 | p<0,05 |
| Dop. | 15 | 163 | 77 | 12 | |
| Dop. | 0 | 110 | 52 | 12 | p<0,001 |
| Dop. | 30 | 210 | 70 | 12 | |
| Dop. | 0 | 110 | 52 | 12 | p<0,001 |
| Dop. | 60 | 291 | 68 | 12 | |
| Dop. | 0 | 110 | 52 | 12 | p<0,001 |
| Dop. | 90 | 250 | 50 | 12 | |
| Dop. | 0 | 110 | 52 | 12 | p<0,001 |
| Dop. | 120 | 211 | 48 | 12 | |
| (MW=Mittelwert, Str.=Streuung, N=Anzahl der Patienten, P(T)=Signifikanz für p<0,05). | | | | | |

Die statistische Auswertung der Dopaminwerte ergibt, daß es 30 min nach Verabreichung von 25 mg Captopril zu einem hochsignifikanten Anstieg von Dopamin kommt.

Tabelle 2 zeigt die statistische Auswertung nach Gabe von 25 mg einer N-(Mercaptoacyl)-aminosäure.

Die statistische Auswertung der gonadotropen Wirkung, die durch die Bestimmung der Hypophysenhormone FSH (Follikelstimulierendes Hormon) und LH (Luteotropes Hormon) möglich ist, ergibt, daß es 30 min nach Verabreichung von 25 mg N-(Mercaptoacyl)-aminosäure zu einem signifikanten Anstieg von FSH und LH kommt.

Die Bestimmung von FSH und LH wurde radioimmunologisch mit Hilfe eines Doppelantikörpers, der mit 125-Jod markiert war (Firma Serono), bestimmt. Die Blutabnahmen wurde vorgenommen wie oben beschrieben. Zu den angegebenen Zeiten wurde bei den Probanden je 5 ml Blut entnommen.

**Tabelle 2**

| Gonadotropes Hormon | Zeit | MW (ng/ml) | Str. | N | P(T) |
|---|---|---|---|---|---|
| LH | 0 | 6,1 | 3,2 | 12 | p<0,05 |
| LH | 30 | 9,3 | 3,1 | 12 | |
| FSH | 0 | 6,8 | 4,7 | 12 | p<0,001 |
| FSH | 30 | 7,9 | 4,5 | 12 | |
| (MW=Mittelwert, Str.=Streuung, N=Anzahl der Patienten, P(T)=Signifikanz für p<0,05). | | | | | |

Anhand der Figuren wird die Erfindung näher erläutert:

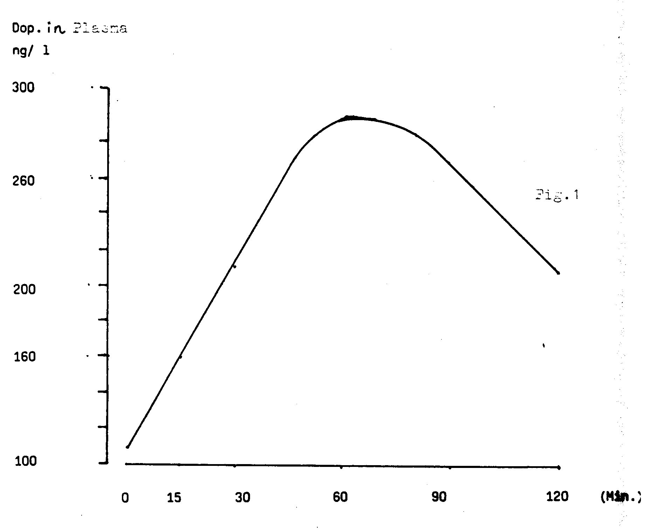

Fig. 1 zeigt den zeitlichen Verlauf der Dopaminfreisetzung nach Verabreichung einer N-(Mercaptoacyl)-aminosäure. Auf der Abszisse ist die Zeit in Minuten und auf der Ordinate der Dopamingehalt im Plasma in ng/l aufgetragen. Die Dimensionsangaben betragen für Dopamin ng/ml.

Es ist ersichtlich, daß es nach oraler Verabreichung von 25 mg Captopril pro Patient (Durchschnittsgewicht 60 kg) bereits nach 15 Minuten zu einem signifikanten Anstieg von Dopamin kommt, der nach etwa 60 min seinen Höhepunkt erreicht und dann wieder kontinuierlich abfällt.

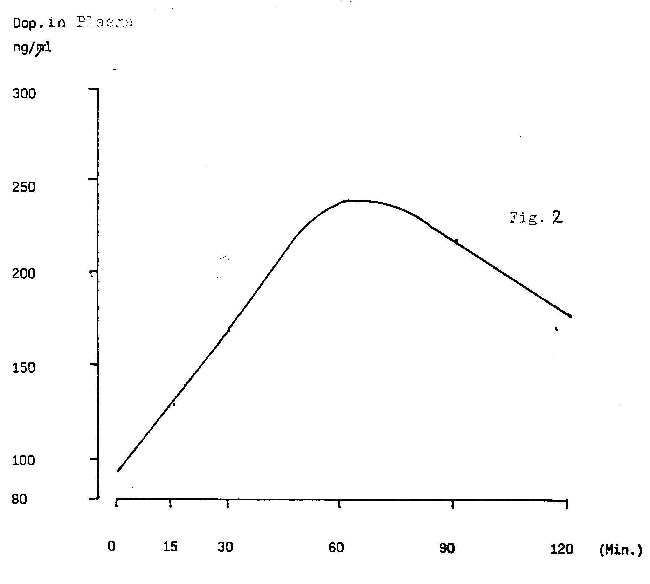

In Fig. 2 ist der zeitliche Verlauf der Dopaminfreisetzung nach Verabreichung von 12,5 mg Captopril graphisch dargestellt. Der Anstieg ist nach 30 min bereits signifikant und erreicht nach 60 min ein Maximum.

## Patentansprüche

1. Verwendung von N-(Mercaptoacyl)-aminosäuren und deren Salzen zur Herstellung von dopaminergen und gonadotropen pharmazeutischen Zubereitungen und Arzneimitteln.

2. Verwendung von N-(Mercaptoacyl)-aminosäuren und deren Salzen nach Anspruch 1 zur Behandlung von Krankheiten mit zerebraler und peripherer Durchblutungsminderung.

3. Verwendung von (2R,4R)-2-(2-Hydroxyphenyl)-3-(3-mercaptopropionyl)-4-thiazolidincarbonsäure nach Anspruch 1 und 2.

4. Verwendung von 1-[(2S)-3-Mercapto-2-methylpropionyl]-2-prolin nach Anspruch 1 und 2.

## Claims

1. The use of N-( mercaptoacyl)-amino acids and their salts for the manufacturing of dopaminergic, prolactin-lowering, gonadotropic pharmaceutical preparations and drugs.

2. The use of N-( mercaptoacyl)-amino acids and their salts as claimed in claim 1 for the manufacturing of drugs for the treatment of illnesses involving a decrease in cerebral and perpheral blood flow.

3. The use of (2R,4R)-2-(2-hydroxyphenyl)-3-(3-aminopropionyl)-4-thiazolidine carbonic acid as claimed in claims 1 and 2.

4. The use of 1-[(2S)-3-amino-2-alkyl-propionyl]-2-proline as claimed in claims 1 and 2.

## Revendications

1. Utilisation de N-(mercaptoacyl)-acides aminés et de leurs sels pour la production de préparations pharmaceutiques dopaminergiques et gonadotropiques et de médicaments.

2. Utilisation de N-(mercaptoacyl)-acides aminés et de leurs sels conformément à la revendication 1 pour le traitement de maladies avec insuffisance vasomotrice périphérique et cérébrale.

3. Utilisation de (2R,4R)-2-(2-hydroxyphényle)-3-(3-mercaptoproplonyle)-4-acides carboxyliques de thiazolidine conformément à la revendication 1 et 2.

4. Utilisation de 1-[(2S)-3-Mercapto-2-méthyle-propionyl] -2-proline conformément à la revendication 1 et 2.
